# EUROPEAN PATENT SPECIFICATION

(11) **EP 3 960 739 B1**
(45) Date of publication and mention of the grant of the patent: **12.11.2025**
(21) Application number: 20729177.4
(22) Date of filing: 23.04.2020
(51) Int. Cl.: C07D 413/12, C07C 233/81, A61P 25/28, A61P 35/00, A61K 31/24, A61K 31/4709

(54) **GRK2 PROTEIN INHIBITOR COMPOUND AND PHARMACEUTICAL COMPOSITION COMPRISING SAME**
GRK2-PROTEIN-INHIBITOR-VERBINDUNG UND PHARMAZEUTISCHE ZUSAMMENSETZUNG DAMIT
COMPOSÉ INHIBITEUR DE LA PROTÉINE GRK2 ET COMPOSITION PHARMACEUTIQUE QUI LE COMPREND

(30) Priority: 26.04.2019 AR P190101103
(43) Date of publication of application: 02.03.2022
(73) Proprietor: Universidad de Buenos Aires, Ciudad Autonóma de Buenos Aires 1053 (AR); Universidad Nacional de Quilmes, Bernal - Prov. de Buenos Aires B1876BXD (AR); Consejo Nacional de Investigaciones Científicas y Técnicas (CONICET), Ciudad Autónoma de Buenos Aires C1425FQB (AR); Lorenzano Menna, Pablo, Bernal - Prov. de Buenos Aires, 1876 (AR)
(72) Inventor: FERNÁNDEZ, Natalia C., Ciudad Autónoma de Buenos Aires, 1228 (AR); ECHEVERRIA, Emiliana, El Talar -Prov. de Buenos Aires (AR); JURITZ, Ezequiel, Quilmes -Prov. de Buenos Aires, 1876 (AR); MONCZOR, Federico, Ciudad Autónoma de Buenos Aires, 1228 (AR); DAVIO, Carlos A., Gral. Rodríguez -Prov. de Buenos Aires (AR); SHAYO, Carina C., Ciudad Autónoma de Buenos Aires, 1426 (AR); RAMIREZ, Javier A., Ciudad Autónoma de Buenos Aires (AR); ACEBEDO, Sofía L., Banfield -Prov. de Buenos Aires, 1828 (AR); LORENZANO MENNA, Pablo, Bernal -Prov. de Buenos Aires, 1876 (AR)
(74) Representative: Schiweck Weinzierl Koch Patentanwälte Partnerschaft mbB
(86) International application number: PCT/IB2020/053874
(87) International publication number: WO 2020/217216

(56) References cited:
- WO-A1-2007/008926
- DATABASE REGISTRY [online] CHEMICAL ABSTRACTS SERVICE, COLUMBUS, OHIO, US; 23 December 2019 (2019-12-23), XP002799510, Database accession no. 0532305365
- MANUELA GUCCIONE ET AL: "G-Protein-Coupled Receptor Kinase 2 (GRK2) Inhibitors: Current Trends and Future Perspectives", JOURNAL OF MEDICINAL CHEMISTRY, vol. 59, no. 20, 8 July 2016 (2016-07-08), US, pages 9277 - 9294, XP055577456, ISSN: 0022-2623, DOI: 10.1021/acs.jmedchem.5b01939

## Description

The present invention relates to a GRK2 protein inhibitor compound. In particular, the invention relates to a GRK2 protein inhibitor compound of Formula I 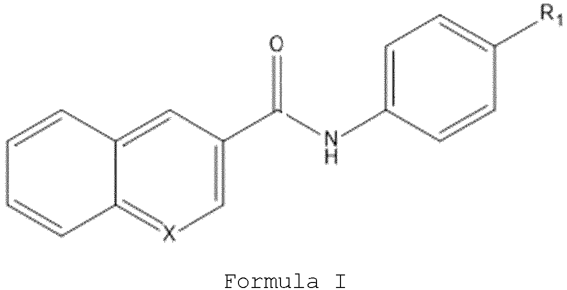
wherein **X** is an atom selected from carbon and nitrogen, and
**R₁** is selected from methyl carboxylate and 1,3,4-oxadiazol-2-yl groups and wherein the compound is selected from methyl 4-(naphthalene-2-amide)benzoate and N-[4-(1,3,4-oxadiazol-2-yl)phenyl]quinoline-3-carboxamide. In a preferred embodiment, the GRK2 protein inhibitor compound is methyl 4-(naphthalene-2-amido)benzoate.

### Background of the invention

The GRK2 protein is a key protein kinase in regulation of a large family of membrane receptors known as *"G protein-coupled receptors"* (GPCR). The main function described for GRK2 is physiological inhibition of the receptors, once they have been steadily stimulated. This phenomenon is known as "desensitization" and it allows cells to dynamically inactivate GPCR-dependent signaling pathways if necessary. The GRK2 protein is involved in the regulation of GPCRs related to cellular responses to adrenaline, histamine, serotonin, acetylcholine, and angiotensin, among others. Thus, excess GRK2 activity was associated with numerous pathologies, and obtaining GRK2 inhibitors is of interest for the pharmacological treatment of these pathologies. In particular, many efforts have been made in the search for GRK2 inhibitors for the treatment of cardiac diseases in which this kinase is known to be overactivated. However, the search for inhibitors has always been oriented to identifying compounds capable of blocking GRK2-mediated kinase or phosphorylation activity of receptors. Today, it is known that the GRK2 protein can not only phosphorylate receptors, but also inactivate them through its RGS domain by blocking the interaction between the receptor and the G-protein through which it signals or transfers the stimulus into the cell, to elicit a biological response. The present invention consists of the development of a new class of compounds capable of inhibiting the action of the RGS domain of GRK2 with high specificity and selectivity.

Although GRK2 protein kinase has been proposed as a target for the development of pharmacological inhibitors, to date only compounds that act by inhibiting the GRK2 kinase activity (of phosphorylation of other proteins) have been proposed. Guccione et al., 2016, discloses a review over the most potent and selective GRK2 inhibitors that have been developed at that time. WO 2007/008926 A1 discloses isoquinoline compounds that influence, inhibit or reduce the action of a G-protein receptor kinase as well as pharmaceutical compositions including therapeutically effective amounts of the isoquinoline compounds and pharmaceutically acceptable carriers and various methods using the compounds and/or compositions to affect disease states or conditions such as cancer, osteoporosis and glaucoma. However, to date, no compounds that inhibit the RGS (or domain) activity of GRK2 (signaling regulator through G protein) have been proposed

### Brief description of the invention

The present invention relates to a GRK2 protein inhibitor compound. In particular, it relates to a GRK2 protein inhibitor compound that of Formula I 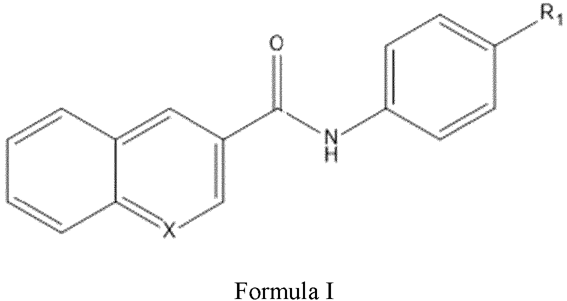
wherein **X** is an atom selected from carbon and nitrogen, and
**R₁** is selected from groups methyl carboxylate and 1,3,4-oxadiazol-2-yl and wherein the compound is selected from methyl 4-(naphthalene-2-amido)benzoate and N-[4-(1,3,4-oxadiazol-2-yl)phenyl]quinoline-3-carboxamide. In a preferred embodiment, the GRK2 protein inhibitor compound is methyl 4-(naphthalene-2-amido)benzoate.

### Brief description of the figures

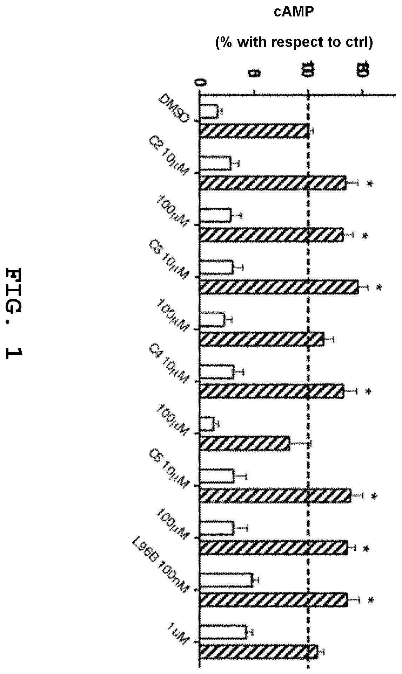

**Figure 1** shows the enhancing effect of comparative compounds C2, C3, C4 and C5 and of compound **L96B** of the invention on the capacity of response to histamine of H2 receptor, a GPCR associated with the Gαs-adenylate cyclase-cAMP signaling cascade that is desensitized by GRK2, in HEK293 cells that overexpress rH2.

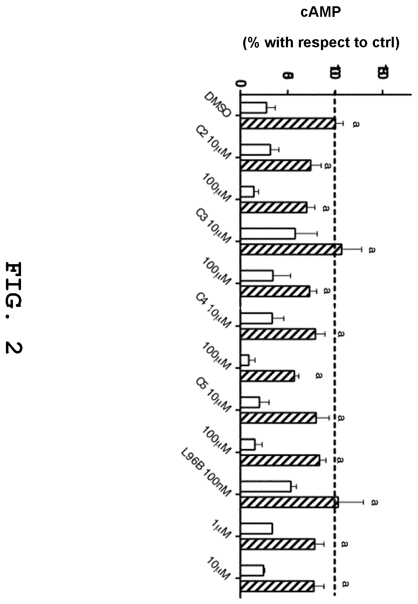

**Figure 2** illustrates the effect selectivity of the inhibitors of the present invention. The figure shows the lack of effect of comparative compounds C2, C3, C4, C5 and of compound **L96B** according to the invention over rH2 capacity of response when the effect is assessed on HEK293 cells that express a variant of GRK2 which has a mutated RGS domain.

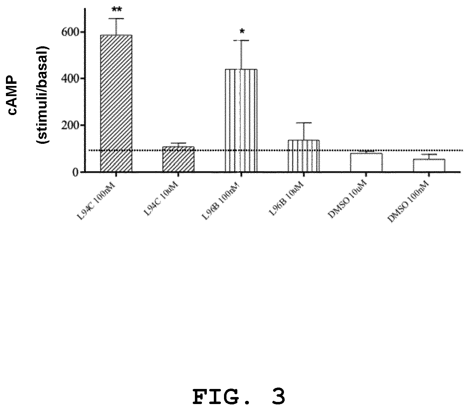

**Figure 3** shows the enhancing effect of the compounds of the invention in a 100 nM concentration on the capacity of response to histamine of H2 receptor, a GPCR associated with the Gαs-adenylate cAMP-cyclase signaling cascade that is desensitized by GRK2, in U937 cells that endogenously express rH2.

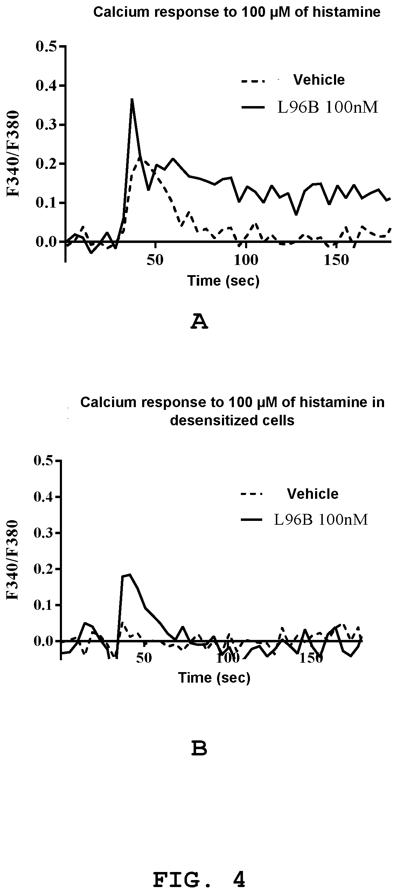

**Figures 4A** and **4B** illustrate the inhibitor enhancing effect on the response of a receptor associated to Gαq-phospholipase C-Ca²⁺ signaling pathway. The figures show that in the A549 cell line, release of Ca²⁺ in response to rH1 stimuli is higher when the cells were treated with compound **L96B** according to the invention.

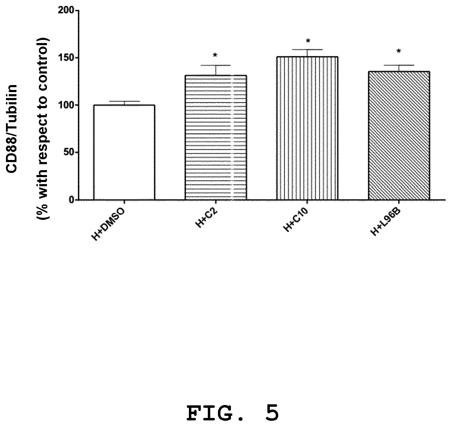

**Figure 5** illustrates the inhibitor effect on leukemic cell differentiation. The figure shows that treatment with a compound according to the invention significantly increases the expression of the CD88 monocyte terminal differentiation marker of a leukemic cell line by inhibiting desensitization by GRK2 of rH2 and, thus, enhancing cAMP response to histamine.

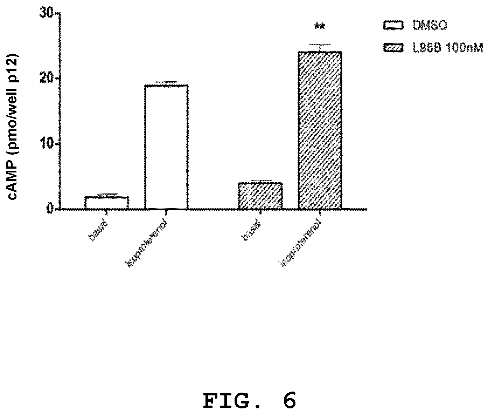

**Figure 6** illustrates the effect of **L96B** on the AMPc response of cardiomyocytes stimulated with the β-adrenergic agonist, Isoproterenol. The figure shows the potentiating effect of compound **L96B** according to the invention on the response capacity of cAMP of β-adrenergic receptors to isoproterenol in rat cardiomyocytes, whose activity is essential for cardiac functioning.

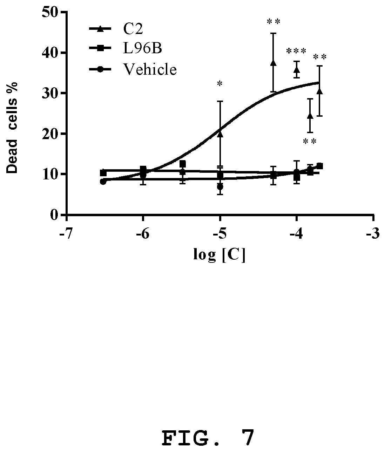

**Figure 7** illustrates the effect of the compounds of the present invention on the viability of immortalized human hepatocytes. It can be observed in **Figure** 7 that comparative compound C2 showed significant cytotoxicity with respect to control with DMSO as from 10 µM concentration. However, compound **L96B** according to the invention did not affect cell viability in the tested concentrations. Thus, there is an advantage of compound **L96B** of the invention with respect to comparative commercial compound C2, related to potential toxic effects over human liver.

### Detailed description of the invention

Since GRK2 is a protein with great physiological relevance and which participates in the inactivation of numerous receptors, its deregulation is associated with the development of various pathologies such as hypertension, cardiac insufficiency, Alzheimer's disease, rheumatoid arthritis, cystic fibrosis and cancer, among others. In all cases, the signaling of a receptor (different for each pathology) is altered due to the over-activation of GRK2, therefore, inhibition of this protein would result in relief of the pathological case or reversal thereof. Thus, to date, there is a need to develop new GRK2 inhibitors that can be used in the treatment or control of the above-mentioned pathologies.

However, there are numerous suppliers who provide molecular tools to laboratories conducting research in the biomedical sciences and who may also be interested in marketing these molecules for *in vitro, in vivo* or *ex vivo* experimental testing where inhibiting the GRK2 RGS domain is useful.

Thus, the present invention refers to GRK2 protein inhibitor compounds. In particular, it refers to a GRK2 protein inhibitor compound of Formula I 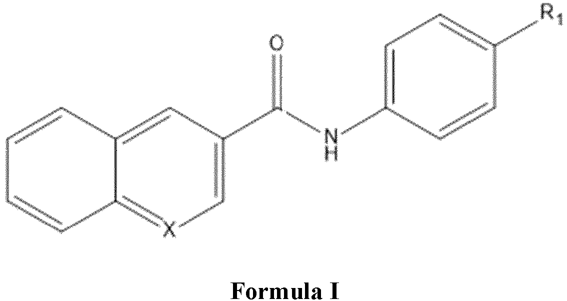 wherein **X** is an atom selected from carbon and nitrogen, and **R₁** is selected from the methyl carboxylate and 1,3,4-oxadiazol-2-yl groups. In a particular manner, the compound of the invention is selected from methyl 4-(naphthalene-2-amido)benzoate and N-[4-(1,3,4-oxadiazol-2-yl)phenyl]quinoline-3-carboxamide. In a preferred embodiment, the GRK2 protein inhibitor compound is methyl 4-(naphthalene-2-amido)benzoate.

In a particular embodiment, the compound of the invention is of Formula **II**

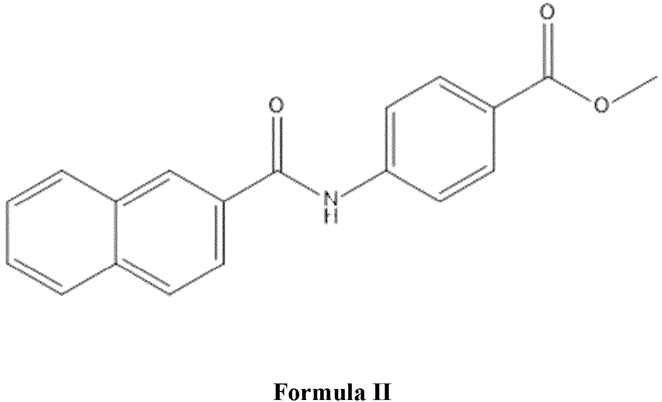

In another particular embodiment, the compound of the invention is of Formula **III**

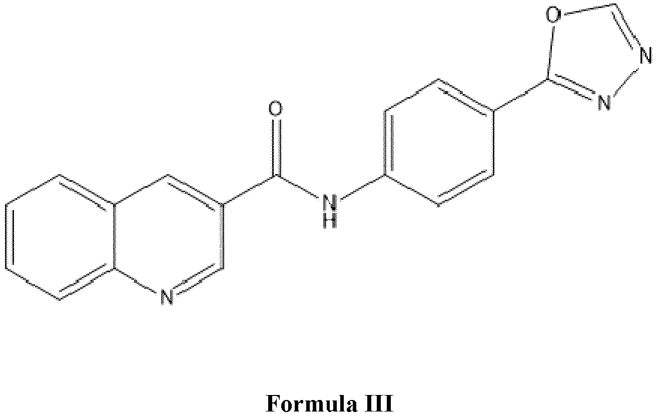

Another object of the invention is a pharmaceutical composition comprising at least a GRK2 protein inhibitor compound and pharmaceutically acceptable excipients. In a particular embodiment of the invention, the pharmaceutical composition comprises the methyl 4-(naphthalene-2-amido)benzoate compound and pharmaceutically acceptable excipients. In another particular embodiment of the invention, the pharmaceutical composition comprises the N-[4-(1,3,4-oxadiazol-2-yl)phenyl]quinoline-3-carboxamide compound. The expert in the art will be able to choose the most appropriate excipients for the composition of the invention, according to the chosen route of administration. Moreover, those pharmaceutical compositions comprising one or more of the compounds of the invention in combination with other therapeutically active substances are considered to be within the scope of the present invention.

Particularly, it is contemplated that the pharmaceutical composition of the invention may be administered by oral, parenteral or transdermal route. In particular, said composition may be in the form of a liquid, a suspension, a tablet, a capsule, a pill, a solution for injection or a transdermal patch. Also contemplated within the scope of the invention is formulating the composition of the invention so as to release in a controlled manner at least one of the compounds with inhibitory activity of a GRK2 cell protein that of formulas I, II and III. The expert in the art will be able to formulate the composition of the invention according to the chosen route of administration.

The present invention also encompasses the pharmaceutical compositions comprising, at least, a compound of Formula I, II or III in combination with other therapeutically active substances, which, along with the compounds of the invention, may provide a therapeutic addition or synergistic effect such as, for example, gemcitabine, capecitabine, decitabine, paclitaxel and docetaxel or other anti-tumor agents.

In another particular embodiment of the invention, the pharmaceutical composition comprises the therapeutically active substance(s) encapsulated in liposomes or microspheres. The expert in the art will be able to establish the necessary parameters and excipients, taking into account the available literature related to this type of encapsulation.

Moreover, another object of the invention is a compound of the invention for use in the treatment of a condition, mediated by a GRK2 cell protein, comprising administering to a patient in need of said treatment a safe and effective amount of at least a compound of the invention.

In another embodiment of the invention, the described inhibitors may be used in basic research as pharmacological tools to promote the understanding of biological, physiological and pathological processes involving GRK2 through the RGS domain thereof, such as mechanisms of desensitization of various GPCRs. In this way, they will allow to identify and assign differential functions to the different GRK2 domains in *in vitro, in vivo* or *ex vivo* experimental assays.

The compounds of the invention may be of interest for the treatment or prevention of cardiac insufficiency (CI), heart hypertrophy and hypertension, as well as for cardiovascular complications in individuals with type 2 diabetes. CI is characterized by an elevated activity of the sympathetic nervous system. The resulting high levels of catecholamines have been associated with increased expression and activity of GRK2 in various tissues. In the heart, this overexpression leads to increased desensitization of GPCRs such as the β-adrenergic and angiotensin II receptors, which regulate contractility and blood flow to the body, respectively. Also, during myocardial ischemia, an increase in membrane GRK2 activity was observed, contributing to the inactivation of the β-adrenergic receptor. Cardiac performance has been shown to increase after inhibition of GRK2 activity. GRK2 has also been shown to play a central role in vasoconstrictor-mediated regulation of arterial tone. Increase in blood pressure induced by GRK2 increases is accompanied by vascular thickening and cardiac hypertrophy. In this sense, inhibition of GRK2 activity may reduce the alterations observed in the vasculature of hypertensive patients.

Moreover, the compounds of the invention could be used for the treatment of different types of cancer, in which an association between GRK2 and the development of cancer is determined. In this regard, the expression of a peptide inhibitor of GRK2, derived from its terminal carboxyl end (βARKct) showed a delay in the growth of prostate tumor cells *in vitro* and prevented the formation of prostate tumors *in vivo* in xenotransplant models.

Furthermore, GRK2 was found to be overexpressed in samples from patients with pancreatic ductal adenocarcinoma (PDAC). The study of GRK2 expression in various breast cancer-derived cell lines, in mammary glands from animal models and in patient samples, showed increased levels of said protein. In this context, xenotransplantation of breast tissue cancer cells that overexpress GRK2 generated larger and earlier tumors with respect to control, while inhibition of its expression prevented the formation of breast tumors in such models. As regards ovarian cancer, high expression of GRK2 was observed in the KGN cell line, derived from granulosa tumor cells, and also in primary and metastatic tumors of patients. This is consistent with the desensitizing function of GRK2 on the follicle-stimulating hormone receptor, responsible for modulating the proliferation and differentiation of granulosa cells and involved in the development of ovarian cancer, suggesting that exacerbated desensitization of this receptor may be part of the pathogenesis of ovarian cancer. In relation to acute myeloid leukemia, it has been demonstrated that the balance between production, degradation and exclusion of cAMP is essential for cell proliferation/differentiation. An increase in intracellular cAMP levels is desirable for differentiation therapy. Thus, histamine stimulation (acting through the H2/Gs receptor) in conjunction with GRK2 inhibitors would increase intracellular cAMP levels to induce the desired differentiation.

In addition, Alzheimer's disease (AD) is a neurodegenerative disorder in which altered expression of GRK2 is an early event in its pathogenesis and precedes the cognitive alterations that characterize the disease. The protein and mRNA levels of GRK2 have been increased in post-mortem hippocampuses and lymphocytes of patients with AD as well as in rat models with chronic cerebral hypoperfusion. Although the signaling pathways that would be altered have not yet been fully elucidated, GRK2 is known to participate in the desensitization of metabotropic glutamate receptors 1 and 5 and the M1 muscarinic receptor, which are involved in the development of AD. Thus, it is anticipated that the described GRK2 inhibitors may result in a therapeutic option.

Furthermore, the GRK2 protein constitutes an important regulator of the responses of the cells of the immune system to situations of inflammation, since it phosphorylates numerous receptors of chemokines and chemotactic responsible for the traffic of leukocytes towards the inflammatory focuses and the exit of T cells from the lymphoid organs. Although the pathogenesis of rheumatoid arthritis (RA), in its complexity, involves several cell types, synovial fibroblasts are responsible for the development of an invasive phenotype. They produce cytokines, which cause inflammation to persist, and proteases that destroy cartilage. It has been shown that during the development of the inflammatory process in rat models of CIA (collagen-induced arthritis), GRK2 levels in synovial fibroblasts increase as the condition progresses, and that GRK2 inhibitors are able to reverse this effect. Additionally, GRK2 inhibition with paroxetine has been shown to attenuate CIA symptoms by reducing T-cell infiltration into synovial tissue.

Results showing a decrease in GRK2 levels in mononuclear blood cells of rheumatoid arthritis have been published in specialized literature.

Moreover, cystic fibrosis (CF) is a disease caused by mutations in the gene that encodes CFTR protein (regulator of the transmembrane conductance of CF) and is characterized by abnormal, excessively thick and viscous secretions that mainly affect the lungs. The CFTR protein is found in the apical membrane of epithelial cells involved in exocrine secretion and acts as a channel for AMP-dependent Cl⁻. Thus, the composition of luminal secretion from the airways is regulated by the activity of the β2 adrenergic receptor (β2AR), which is coupled to the cAMP pathway. In airway samples from cystic fibrosis patients, a reduction in the density of this receptor has been observed, as well as an over-expression of GRK2. Accordingly, the culture of these epithelial cells showed a reduced secretion of Cl⁻ in response to isoproterenol. Therefore, inhibition of GRK2 would be a potential target for increasing the activity of adenylate cyclase in response to activation of the β2 adrenergic receptor.

Additionally, sepsis is a complex clinical syndrome resulting from a host's harmful response to infection. Polymorphonuclear neutrophils (PMN) are the host's first line of defense against microorganisms, being recruited at the inflammatory sites by chemoattractants such as leukotriene B4 (LTB4) and chemokines. Once migrated, these leukocytes are capable of phagocyting and generating large quantities of reactive oxygen and nitrogen species, such as hydrogen peroxide and nitric oxide, which are crucial products for the microbicidal activity of these cells. Since neutrophils play a key role in the control of the infectious process, it can be hypothesized that poor neutrophil migration may aggravate infections. Data suggest that endogenous mediators produced during sepsis may continuously activate circulating neutrophils, leading to the activation of GRK2, which in turn may induce desensitization of neutrophils to chemoattractants. Inhibition of GRK2 may be beneficial by restoring the ability to migrate to neutrophils. Finally, multiple sclerosis (MS) is a chronic inflammatory disease of the central nervous system. The infiltration of activated T-lymphocytes and macrophages into the brain and spinal cord is an important factor in the pathogenesis of this disease. In a mouse model of MS, a more benign course of the disease was demonstrated in GRK2 +/- mice. It was also shown that GRK2 expression is reduced during the remission phase in MS patients, suggesting that the reduction of GRK2 activity during MS may be a new target for the therapy.

Thus, taking into account that the inhibition of GRK2 activity would be beneficial for controlling and/or treating the above mentioned disorders and diseases, the compounds of the invention could be used for controlling and/or treating such disorders and diseases. In particular, the compounds of the invention are useful in the treatment and/or control of heart disease, various types of cancer, inflammatory diseases, Alzheimer's disease (AD), rheumatoid arthritis, cystic fibrosis (CF), sepsis and multiple sclerosis (MS).

A particularly preferred object of the present invention is a pharmaceutical composition comprising a compound of Formula II or a compound of Formula III or one of their salts. However, the invention also includes the pharmaceutical compositions comprising one or more of said compounds of the invention, in combination with one or more second therapeutically active substances.

According to the present invention, the term "administering" and variants thereof (for example, the "administration" of a compound) with reference to a compound of the invention, means to introduce a compound, or a salt of the compound of the invention, into the system of the animal or human in need of said treatment. When a compound of the invention, or a salt of the compound of the invention is provided in combination with one or more other active agents (for example, another therapeutically active substance that presents a complementary or synergistic therapeutic effect to that of the compound of the invention), "administration" and its variants must be, each one, understood as including the sequential and concurrent introduction of the compound, or the salt thereof and of the other agents.

The term "patient" for the purposes of the present invention includes humans. However, it should be understood that the compounds may also be administered to other animals, particularly mammals and other organisms. Thus, a compound according to the present invention is applicable both to human therapy and veterinary applications. Therefore, in some particular embodiments, the compounds are for administration to mammals, and in other applications they are for administration to humans.

According to the present invention, a "pharmaceutically acceptable salt" of a compound means a salt which is pharmaceutically acceptable, and which possesses the desired pharmacological activity of the starting compound. It should be understood that salts are not toxic Additional information about suitable salts can be found in Remington the Science and Practice of Pharmacy 21st ed., Pharmaceutical Press, London, England, 2011. It should also be understood that the compounds of the invention may have one or more salts or one or more pharmaceutically acceptable derivates associated thereto.

By "prodrug" is meant compounds that are transformed (typically rapidly) *in vivo,* to obtain the starting compound of the above formula, for example, by hydrolysis in the blood. The expert in the art will know how to prepare prodrugs of the compounds of the invention in accordance with the appropriate practices to that purpose. They are, however, not part of the invention.

"Therapeutically effective amount of" or "safe and effective amount of" is an amount of a compound of the invention which, when administered to a patient, effectively treats a certain disease. The amount of a compound of the invention constituting a "therapeutically effective amount of" will vary depending upon a number of factors, including activity, metabolic stability, excretion rate and duration of compound action, age, weight, general health condition, sex, diet and species of the patient, route and time of administration of the compound, concurrent administration of adjuvants or additional therapies, and the severity of the disease for which the therapeutic effect is sought. The "therapeutically effective amount" or the "safe and effective amount" for a given circumstance can be determined without the need for extra experimentation. In preferred embodiments, the acceptable dose range is from 10 mg to 500 mg every 24 hours, via oral, intramuscular or intravenous route. As is known in the art, adjustments for systemic versus localized release, the age, weight, general health condition, sex, diet and species of the patient, and route and time of administration of the compound, concurrent administration of adjuvants or additional therapeutically active ingredients, and the severity of the disease for which the therapeutic effect is sought may be necessary, and will be ascertainable through routine experimentation. The effective amounts or doses of the compounds of the present invention may be determined by routine methods, such as modeling, dose escalation or clinical trials, taking into account routine factors. Actual dosage levels of active ingredients in pharmaceutical compositions may vary. In general, an adequate daily dosage of a compound and/or of the compositions according to the present invention will be the amount of the compound that provides the lowest dose that is effective and produces the desired therapeutic effect.

The compounds of the invention can be administered to a patient by any acceptable route of administration. Acceptable routes of administration include, but are not limited to, oral, parenteral, transdermal, endocervical, endosinusal, enteral, intra-abdominal, intra-arterial, intrabronchial, intracerebral, intracoronary, intradermal, intraductal, intraduodenal, intradural, intraepidermal, intraesophageal, intragastric, intragingival, intraileal, intralymphatic, intrameninge, intramuscular, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal, nasogastric, buccal, percutaneous, peridural, rectal, respiratory (inhalation), subcutaneous, sublingual, submucosal, topical, transmucosal, transtracheal, ureteral, urethral and vaginal.

The compounds of the invention may be administered in any acceptable solid, semisolid, liquid or gaseous dosage form. Acceptable dosage forms include, but are not limited to, tablets, capsules, solutions, sprays, creams, emulsions, gases, gels, grains, liniments, lotions, suppositories, ointments, pastes, powders, suspensions, syrups and tablets.

A dosage form of the invention may comprise only one compound of the invention, or the compound of the invention may be formulated together with conventional excipients, pharmaceutical carriers, adjuvants and/or other medicinal or pharmaceutical agents. Acceptable excipients include, but are not limited to, (a) anti-adherents, such as sodium croscarmellose, crospovidone, sodium glycolate starch, microcrystalline cellulose, starch and talc; (b) binders, such as cellulose, gelatin, hydroxypropyl cellulose, lactose, polyethylene glycol, polyvinylpyrrolidone, sorbitol, starch and xylitol (c) coatings, such as cellulose and shellac; (d) disintegrants, such as cellulose, polyvinylpyrrolidone, sodium carboxymethyl cellulose, methylcellulose, microcrystalline cellulose and sodium starch glycolate and starch; (e) fillers such as calcium carbonate, cellulose, dibasic calcium phosphate and mannitol; (f) flavoring agents/aromatizers; (g) coloring agents; (h) glidants such as calcium stearate and colloidal silicon dioxide; (i) lubricants, such as calcium stearate, magnesium stearate, polyethylene glycol, and talc; and (j) preservatives, such as citric acid, vitamin C and vitamin E. Pharmaceutical carriers include soluble polymers, microparticles made of polymers, natural or synthetic, insoluble or biodegradable, microcapsules, lipoproteins, liposomes, and micelles.

A pharmaceutical composition of the invention will include a therapeutically effective amount of a compound of the invention, or a pharmaceutically acceptable salt thereof, with the rest of the pharmaceutical composition being composed by one or more pharmaceutically acceptable excipients. Generally, a compound of the invention, or a pharmaceutically acceptable salt thereof, will be in a proportion from 1% to 99% by weight of the pharmaceutically acceptable composition, with the rest of the pharmaceutical composition being composed of one or more pharmaceutically acceptable excipients. Typically, a compound of the invention, an individual stereoisomer thereof or a mixture of stereoisomers thereof, a prodrug, a derivative or a pharmaceutically acceptable salt thereof, will be in a proportion from 5% to 75% by weight of the pharmaceutically acceptable composition, with the rest of the pharmaceutical composition being composed of one or more pharmaceutically acceptable excipients. Methods to prepare the dosage forms of the invention are known or will be apparent to those experts in the art; see, for example Remington the Science and Practice of Pharmacy 21st ed. (Pharmaceutical Press, London, England, 2011).

A therapeutically effective amount of a compound of the invention will vary depending on a series of factors, including activity, metabolic stability, excretion rate and duration of compound action, age, weight, general health condition, sex, diet and species of the patient, route and time of administration of the compound, presence of adjuvants, of additional therapeutically active ingredients in the composition, and the severity of the disease for which the therapeutic effect is sought.

The present invention also encompasses the procedures to be used for the obtention of the compounds of Formula I, II and III of the invention and intermediary compounds to be used in said procedure.

### Examples

### Example 1

### Synthesis of methyl 4-(2-naphthamide)benzoate (L96B)

Step a: 4.0 g (29.2 mmol) of 4-aminobenzoic acid were dissolved in 40 mL of MeOH. This solution was cooled down to 0°C, 2,5 equivalents of SOCl₂ were added dropwise and it was refluxed for 2.5 h, under vigorous stirring. Solvent was then evaporated, it was neutralized with NaHCO₃ saturated solution (s.s.) and the mixture was extracted with EtOAc. The organic phase was washed with NaCl s.s., dried out over anhydrous Na₂SO₄ and the solvent was evaporated at reduced pressure. Purification of the raw residue by column chromatography (n-hexane/EtOAc) allowed to obtain methyl 4-aminobenzoate with a 70% yield (3.1 g).

Step b: Afterwards, 500 mg (3.31 mmol) of the product of Step a were dissolved in CH₂Cl₂, triethylamine was added (3 equivalents) and 569 mg (3.31 mmol) of 2-naftoic acid were added to this solution. Then, SOCl₂ was added dropwise at room temperature under vigorous stirring, until completing the reaction. Solvent was evaporated at reduced pressure, residue was dissolved in CH₂Cl₂ and washed with 1 N HCl, NaCl s.s., 1 N NaOH and finally with NaCl s.s., it was dried out over anhydrous Na₂SO₄ and the solvent was evaporated at reduced pressure. Purification of the raw residue by column chromatography (CH₂Cl₂) allowed to obtain **methyl 4-(2-naphthamide)benzoate (L96B)** with a 69% yield (700 mg).
M.p.: 142°C
MS (ESI) m/z for M+H⁺:291.0890.
RMN-¹H (500 MHz, CDCl₃): 3·92 (CH3, 3H, s); 7.57-7.63 (2 H, m); 7.80 (2 H, d, J = 8.7 Hz); 7.90-7.93 (2 H, m); 7.95 (2 H, t, J = 8.2 Hz); 8.08 (2 H, d, J = 8.7 Hz); 8.16 (NH, 1 H, s); 8.39 (1 H, s).
RMN-¹³C (125 MHz, CDCl₃): 52.2 (CH3); 119.4; 123.5; 126.0; 127.3; 127.9; 128.0; 128.3; 129.1; 129.2; 131.1; 131.8; 132.7; 135.2; 142.3; 166.0 (CO amide); 166.7 (CO ester).

### Example 2

### Synthesis of N-[4-(1,3,4-oxadiazol-2-yl)phenyl]quinoline-3-carboxamide (L94C).

Methyl 4-(quinoline-3-carboxamide)benzoate (L94A) was obtained following a procedure similar to that described in Example 1, but using 3-quinolincarboxilic acid in Step b.

The compound obtained (200 g, 0.66 mmol) was dissolved in MeOH, 5 mL of hydrazine hydrate solution were added and left to stir at r.t. until the reaction was complete. The solvent was evaporated at reduced pressure. Purification of raw residue by CH₂Cl₂ crystallization allowed to obtain N-(4-(hydrazinecarbonyl)phenyl)quinoline-3-carboxamide with a 50% yield (100 mg). Finally, 25 mg (80 µmol) of the product obtained in the previous step were suspended in 3 mL of trimethyl orthoformate and it was heated to reflux under vigorous stirring until the reaction was complete. Solvent was evaporated at reduced pressure. Purification of raw residue by washing with ethyl ether and further recrystallization with CH₂Cl₂ allowed to obtain **N-[4-(1,3,4-oxadiazol-2-yl)phenyl]quinoline-3-carboxamide (L94C)** with a 15% total yield.
M.p.: 202°C
MS (ESI) m/z for M+H⁺:317.10390.
RMN-¹H (300 MHz, DMSO-d6):7.65-7.82 (7H, m); 8.20 (1H, d, J = 7.9 Hz); 8.37 (1H, d, J = 7.9 Hz); 8.73 (1H, s); 9.25 (1H, s); 9.51 (NH, 1H, s).
RMN-¹³C (75 MHz, DMSO-d6): 123.3; 123.3; 123.6; 125.1; 126.1; 127.1; 127.1; 128.5; 128.7; 128.9; 131.6; 137.4; 143.0; 145.8; 149.9; 151.0; 162.2; 163.9.

### Example 3

### Enhancing effect of inhibitors over the response of a receptor associated to the Gαs-adenylate-cyclase-cAMP route

Inhibiting activity of the compounds of the invention and of the following compounds from the Enamine library was studied:

### COMPOUNDS ACCORDING TO THE INVENTION

**L96B:** methyl 4-(naphthalene-2-amido)benzoate
**L94C:** N-[4-(1,3,4-oxadiazol-2-yl)phenyl]quinoline-3-carboxamide

### TESTED COMPOUNDS FROM THE ENAMINE LIBRARY

| | |
|---|---|
| **C4:** | 3-(naphtalen-2-yl)-1-[3-(1H-pyrazol-1-yl) piperidin-1-yl]propan-1-one |
| **C2:** | 3-(1-acetyl-2,3-dihydro-H-indol-5-yl)-1-(quinoline-8-yl)urea |
| **C3:** | 2,7-dimethyl-N-[3-(1,3,4-oxadiazol-2-yl)phenyl] quinoline-3-carboxamide |
| **C5:** | 2-(4-oxo-3,4-dihydrophthalazin-1-yl)-N-(quinoline-5-yl)acetamide |
| **C10:** | N-(2,3-dihydro-1H-inden-1-yl)-3-[5-(4-methylphenyl)-1,3,4-oxadiazol-2-yl]propanamide |

In order to assess the effect of inhibitors of the RGS domain of GRK2, a cell model derived from HEK293 transfected cells was obtained. Such cells overexpress the histamine H2 receptor (rH2), a G-protein coupled receptor (GPCR) of the Gs type that, when activated, promotes cAMP synthesis and which is inactivated by regulatory domain-mediated desensitization of G-protein stimulation (RGS) of GRK2. The clone obtained not only overexpresses rH2 but also overexpresses a negative dominant mutant of GRK2, the GRK2-K220R variant, which cannot phosphorylate GPCRs but can inactivate them through its RGS domain. In this system, the desensitizing effect of the RGS domain of GRK2 on the cAMP response of rH2 is evident. When pre-treated with a compound, an enhancing effect of cAMP response to the agonist may be associated with an inhibition of RGS-domain mediated desensitization of GRK2.

Clones derived from HEK293 cells were obtained by stable and sequential transfection with pcDNA3.1Zeo(+)-HARH2 and pcDNA3-GRK2K220R plasmids using K2 Transfection System (BIONTEX) reactive, following manufacturer's instructions and using, for a 35 mm diameter plate, a mixture A of 2.7 µg of total DNA and 150 µl of DMEM base and a mixture B of 6 µl of K2 transfection reactive and 150 µl of base DMEM. The following day, cells were plated in 96-well plates with 0.3 ml of complete DMEM per well. For clone selection, cells were incubated in selection agent-supplemented medium (50 µg/ml Zeocine or 1.2 mg/ml geneticin), following a limit dilution protocol. According to this protocol, cells were counted and plated performing serial dilutions, so as to obtain wells with con 1000, 100, 10 and 1 cell. The medium was renewed every other day by replacing 100 µl in each well with 100 µl of fresh medium of the same composition. After three weeks, several antibiotic-resistant clones were selected and afterwards plasmid expression was analyzed by *Western blot.*

Adhered clones were maintained in a humidified-atmosphere oven, with 5% CO₂ at 37°C, in 10% FBS-supplemented DMEM medium, 50 µg/ml gentamicin, 0.8 mg/ml geneticin and 50 µg/ml zeocin. Cells were passed by adding 0.05% trypsin solution and 0.3 mM EDTA.

In order to assess the effect of GRK2 inhibitor compounds, clones were plated in 48-well plates at 50% confluence. After 24 h, cells were pretreated in minimum medium at 37°C for 40 min with commercial compounds C2, C3, C4 and C5, at 10 µM or 100 µM concentrations or with compound **L96B** according to the invention at 100 nM or 1 µM concentrations. An equivalent amount of DMSO vehicle was used as negative control.

Next, cells were incubated for 3 min with 1 mM IBMX, a phosphodiesterase inhibitor that prevents degradation of the cAMP produced and afterwards the rH2-Gαs-adenylate-cyclase-cAMP signaling route was stimulated for 9 minutes with the rH2-specific agonist, amthamine, at a 10 µM concentration. Afterwards, cells were lysed and resuspended in absolute ethanol. This was evaporated and cAMP levels were determined.

cAMP in samples was quantified by adapting the linking protein-binding technique according to Davio et al, 1995.

**Figure 1** shows the enhancing effect of commercial compounds C2, C3, C4 and C5 and of compound **L96B** according to the invention on H2 receptor capacity of response to histamine, a GPCR associated with the Gαs-adenylate-cyclase-cAMP signaling cascade that is desensitized by GRK2. (Plain bars correspond to cAMP basal levels and striped bars correspond to rH2 response to 10 µM of its specific agonist, amthamine. *p<0.05 with respect to cells stimulated without inhibitor)

### Example 4

### Selectivity of inhibitor effect.

In order to assess specificity of the effect of compounds over RGS domain of GRK2, assays were carried out in a second system that overexpresses rH2 and GRK2K220R/R106A, a GRK2 double mutant negative dominant construct that lacks both kinase activity and desensitization action through RGS domain. In this case, the overexpression of the inactive variant of GRK2 prevents regulation of the rH2response. The R106A mutation that inactivates the RGS domain is redundant to the effect of an inhibitory potential, thus constituting the selectivity control of the model and allowing to determine if the enhancement in the receiver response occurred as a result of an inhibition of the RGS domain, or if it was the result of a non-specific action of the compound on an unwanted target. Those compounds that also raise cAMP levels in this control system are considered to be of non-specific action.

**Figure 2** shows the lack of effect of comparative compounds C2, C3, C4, C5 and of compound **L96B** according to the invention over rH2 capacity of response when the effect is assessed in HEK293 cells that express a variant of GRK2 which has a mutation in the RGS domain. (Plain bars correspond to cAMP basal levels and striped bars correspond to rH2 response to 10 µM amthamine, its specific agonist, "a" indicates that there are no significant differences between the mentioned values).

The enhancing effect of compounds C2, C3, C4, C5 and **L96B** does not occur in clones derived from HEK293 cells which stably express rH2 and a double mutant GRK2 variant deficient in kinase activity and RGS activity. Since the latter mutation is redundant to the effect of a potential inhibitor of RGS domain of GRK2, this system allows us to discern whether the enhancement in the rH2 response occurred as a result of an RGS domain inhibition, or whether it resulted from a non-specific action of the compound on an unwanted target.

In order to assess the effect of GRK2-inhibitor compounds over cAMP response under more physiological conditions, cAMP response was determined in U937 cells that were not transfected as they endogenously express rH2.

400,000 cells were starved for two hours without fetal bovine serum, pretreated in minimum medium (RPMI), at 37°C for 40 min, with compounds **L96B** and **L94C** of the invention, at 100 nM and 10 µM concentrations. An equivalent amount of vehicle, DMSO, was used as negative control.

Next, cells were incubated for 3 min with 1 mM IBMX, a phosphodiesterase inhibitor that prevents degradation of cAMP produced. Next, the rH2-Gαs-adenylate-cyclase-cAMP signaling route was stimulated for 9 minutes with the rH2-specific agonist, amthamine, at a 10 µM concentration. Afterwards, cells were lysed and resuspended in absolute ethanol. This was evaporated and cAMP levels were determined.

CAMP in samples was quantified by adapting the linking protein-binding technique according to Davio et al, 1995.

**Figure 3** shows the enhancing effect of the compounds of the invention at 100 nM concentration on H2 receptor capacity of response to histamine, a GPCR associated with the Gαs-adenylate-cyclase-cAMP signaling cascade that is desensitized by GRK2. (Plain bars correspond to cAMP response to rH2 agonist amthamine in DMSO-pretreated cells and striped bars correspond to cAMP response to amthamine agonist in cells pretreated with the compounds. *p<0.05; **p<0.005 with respect to cells stimulated without inhibitor which represent 100%.)

In order to assess the effect of the inhibitors of RGS domain of GRK2 on the response of GPCRs coupled to Gq-PLC-calcium intracellular signaling route, histamine H1 receptor was used as example, a Gq protein coupled receptor, which, upon activation, promotes calcium release from the endoplasmic reticulum into the cellular cytoplasm and is also inactivated by desensitization mediated by RGS domain of GRK2.

The system used for determining this biological activity consists of epithelial lung cell-line, A549, which endogenously express rH1 and GRK2. Calcium cytoplasmic levels were assessed using Fura2AM fluorescent probe, which is capable of binding to Ca²⁺ ion, whereby excitation wavelengths (λ) are altered, varying from 380 nm (unbound) to 340nm (bound). Accordingly, a calcium response by the receptor to stimuli is evidenced by an increase in fluorescence emitted by Fura2AM in its calcium-bound form and a decrease in signal emitted by its unbound form, giving an increase in fluorescence ratio F340/F380.

A549 cells were pleated at 40% confluence in 96-wells plates. The next day, the cells were incubated with FURA 2-AM 2 mM probe and 0.2% pluronic acid in buffer BSS (140 mM NaCl, 3.9 mM KCl, 0.7 mM KH₂PO₄, 0.5 mM Na₂HPO₄, Al₂H₂O, 1 mM CaCl₂, 0.5 mM MgCl₂, and 20 mM HEPES, 10 mM glucose and 0.1% BSA pH=7,5) for 90 min at 37°C. Then, the cells were washed with BSS twice. During the last 40 min of said incubation, the compound **L96B** according to the invention either was added at a 100 nM concentration or was not added at all. Intracellular calcium levels were recorded at 37°C in real time, with a *FlexStation 3* (*MolDev*) equipment alternating excitations at 340 nm and 380 nmen. Fluorescence emission was measured at 505 nm. Histamine ligand at a 100 µM concentration was pipetted in each well at 30 sec after initiating reading and without interrupting the same, and 0.1% Triton x100 detergent was pipetted at 180 sec to estimate the amount of FURA2-AM incorporated by the cells.

In order to assess the response in desensitized cells, A549 cells were plated at 40% confluence in 96-well plates. The following day they were incubated with 2 mM FURA 2-AM and 0.2% pluronic acid, in BSS for 90 min at 37°C. During the last 10 min of said incubation, either histamine was added to the corresponding wells at a 33 µM concentration or only vehicle was added. Next, two BSS washes were performed, and procedure was followed as in response assays.

**Figures 4A** and **4B** show that in the A549 cell line, Ca²⁺ release in response to rH1 stimuli is higher when the cells were treated with the compound **L96B** of the invention. This indicates a lower GRK2-mediated rH1 inactivation. Furthermore, this compound inhibits desensitization of Ca²⁺ response mediated by GRK2 in cells that were pretreated with 33 µM histamine.

### Example 5

### Effect of inhibitors on leukemic cell differentiation

Induction of cell differentiation is a desirable event to achieve cell maturation and slow down leukemic cells proliferation. This process is closely related to the generation of the second cAMP messenger in response to different stimuli. The effect of the compounds on the differentiation of U937 leukemic cells was studied by measuring the expression of the terminal differentiation marker to CD88 monocytes after joint treatment with histamine (an agent that increases cAMP intracellular levels, but due to the fast desensitization of its receptor by GRK2, cAMP signal is insufficient to induce cell differentiation), the compounds C2 and C10 and compound **L96B** according to the invention.

Promonocytic leukemia U937 cells were cultivated in suspension in an oven with humidified atmosphere, with 5% CO₂, at 37°C, in 10% FBS-supplemented RPMI 1640 medium and 50 µg/ml gentamicine, and maintained at a density of 4x10⁵-1.6x10⁶ cells/ml. U937 cells were treated for 48h with a concentration of 100 µM of histamine and 1 µM of compounds C2 and C10 or with compound **L96B** of the invention.

Samples were lysed in Laemmli buffer (50 mM Tris-HCl pH 6.8; 2% SDS; 100 mM 2-mercaptoetanol; 10% glycerol and 0.05% bromophenol blue), sonicated and incubated in water bath for 5 minutes prior to seeding. Protein extracts were analyzed in polyacrylamide gel at 12%, under denaturalizing conditions (SDS-PAGE). The electrophoresis buffer used consisted in 25 mM Tris; 192 mM glycine; 0.1% SDS, pH 8.3. Electrophoresis was developed in minigels at a constant current of 30 mA (BioRad). After fractioning, the gel was balanced in transfer buffer (25 mM Tris HCl, pH 8.3, 150 mM glycine; 20% methanol) for 15 min and transferred to nitrocellulose membranes at 100 V, for 1 h at 4°C. The proteins transferred to the membranes were stained in a solution of 0.2% Ponceau; 0.5% acetic acid, to visualize the total proteins and to verify the transfer efficiency in all the lanes. Afterwards, the membranes were washed in PBS, until the staining disappeared. After treating the membranes with blocking solution (PBS-0.1% Tween20) for 1 h, they were incubated for 1 h with 1 µg/ml of goat anti-CD88 antibody or rabbit anti-beta-tubulin antibody. Detection was carried out by incubating with 0.2 µg/ml of secondary anti-goat or anti-rabbit antibody conjugated to peroxidase for 1 h, followed by exposure to a peroxidase substrate and chemiluminescence amplifier solution (*Amersham Life Science*). The result was displayed by autoradiography.

The intensity of the band corresponding to CD88 was quantified by optical densitometry using *ImageJ* software and expressed as relative to the value obtained for tubulin. Results were expressed considering the expression of CD88 in the cells treated with the DMSO vehicle as 100%. The data represent the mean ± ESM of two independent experiments (n=2).

**Figure 5** shows that treatment with a compound of the invention significantly increases the expression of the CD88 monocyte terminal differentiation marker by inhibiting desensitization by GRK2 of rH2 and enhancement of cAMP response to histamine. (Plain bars correspond to CD88 levels in cells pretreated with histamine (H) and vehicle (DMSO) and striped bars correspond to CD88 levels in cells pretreated with histamine (H) and compounds. *p<0.05)

### Example 6

### Effect of the compounds of the invention over cAMP response of cardiomyocytes stimulated with β-adrenergic agonist, isoproterenol

B-adrenergic receptors are responsible for controlling myocardial strength and contractility. The functioning of these receptors is regulated by GRK2 and their desensitization by GRK2 is exacerbated in patients with hypertension or cardiac insufficiency.

Cardiomyocytes were obtained from the ventricle of neonatal Sprague-Dawley rats by proteolytic digestion. Briefly, after decapitation, the ventricles were separated and transferred to iced PBS where they were cut with fine scissors into 1-3 mm³ portions. The tissue thus disintegrated was subjected to three rounds of enzymatic digestion in saline solution containing collagenase IV (1 mg/ml). The disintegrated cells were collected by centrifugation at 400 rpm for 5 minutes and kept in DEM-F12 medium containing 10% BFS, 10 µg/ml insulin, 10 µg/ml holo-transferrin, 100 µM bromodeoxyuridine (to avoid the proliferation of other cell types), 10,000 U/ml penicillin and 10 mg/ml streptomycin. The cells were plated in Petri dishes and kept for 1h at 37°C in atmosphere humidified with 5% CO₂. The cells that did not adhere to the plate, corresponding to cardiomyocytes, were transferred at a density of 0.5×10⁶ cells/well to 12-well multi-well plates. All of the experimentation with animals was carried out following the guidelines of the National Institute of Health Guide for the Care and Use of Laboratory Animals (2011) and were approved by the Institutional Committee for the Use and Care of Laboratory Animals of the School of Pharmacy and Biochemistry of the UBA (University of Buenos Aires) (Resolution (D) No. 19-16).

To perform the isoproterenol response tests, the cells were starved for 2h and pretreated for 40 min with compound **L96B** according to the invention at a 100 nM concentration or equivalent amounts of DMSO, in minimum medium, at 37°C. Then, cells were incubated for 3 min with 1 mM phosphodiesterase inhibitor, IBMX, and then stimulated for 9 min with 1 µM isoproterenol to activate the production of cAMP evoked by the β-adrenergic receptors.

Afterwards, cells were lysed and resuspended in absolute ethanol. This was evaporated and cAMP levels were determined. cAMP in samples was quantified by adapting the linking protein-binding technique according to Davio et al, 1995.

**Figure 6** shows the enhancing effect of the compound **L96B** of the invention, over the response capacity of cAMP of β-adrenergic receptors to isoproterenol, whose activity is essential for cardiac functioning. (Plain bars correspond to cAMP response to isoproterenol in DMSO-pretreated cells and striped bars correspond to cAMP response to isoproterenol in cells pretreated with the compound **L96B** according to the invention. **p<0.005 with respect to cells stimulated without inhibitor)

### Example 7

### Effect of the compounds of the invention on viability of immortalized human hepatocytes

Thirty percent of drug development processes are unsuccessful due to unexpected toxicity of a compound. Since hepatotoxicity is one of the main forms of toxicity in which drugs affect the body, the cytotoxicity of inhibitors was assessed using the human hepatocyte HEPG2 cell line.

Cells were plated in a 96-well plate at a 22,000 cell/well density and incubated for 48 hours with the compounds in a concentration range of between 0.33 µM and 200 µM. Afterwards, their viability was analyzed through trypan blue exclusion method and counted in Neubauer camera.

It can be observed in **Figure** 7 that compound C2 showed significant cytotoxicity with respect to control with DMSO as from a 10 µM concentration. **L96B** did not affect cell viability in the tested concentrations. Thus, there is an advantage of compound **L96B** of the invention with respect to comparative commercial compound C2, in relation to potential toxic effects on human liver. EC₅₀ obtained for compound C2 is 10.3 µM ± 3.2; *p<0.05; **p<0.01; ***p<0.005).

### Example 8

### Example of pharmaceutical formulations containing the compounds of the invention

The following examples disclose representative pharmaceutical compositions comprising one or more of the compounds according to the invention.

| Injectable formulation | |
|---|---|
| Compound of **Formula I** or **Formula II** | 10.00 mg |
| Propylene glycol | 800.00mg |
| Ethanol | 252 µL / 10.13 % W/V |
| Sodium benzoate | 95.00mg |
| Benzoic acid | 5.00 mg |
| Benzyl alcohol | 28 µL |
| Water for injectables | q.s. 2 mL |

| Tablet for oral formulation | |
|---|---|
| Compound of **Formula I** or **Formula II** | 10 mg |
| Magnesium carbonate | 43.02 mg |
| Cornstarch | 36.49 mg |
| Colloidal silicon dioxide | 1.50 mg |
| Sodium lauryl sulfate | 2.81 mg |
| Croscarmellose sodium | 3.00 mg |
| Microcrystalline cellulose | 30.40 mg |
| Magnesium stearate | 3.00 mg |
| Pregelatinized starch | 4.78 mg |

### REFERENCES

Akhter, S.A, et al., In vivo inhibition of elevated myocardial beta-adrenergic receptor kinase activity in hybrid transgenic mice restores normal beta-adrenergic signaling and function. Circulation, 1999. 100(6): p. 648-53.
Benovic, J.L., et al., Beta-adrenergic receptor kinase: primary structure delineates a multigene family. Science, 1989. 246(4927): p. 235.
Bookout, A.L., et al., Targeting Gbetagamma signaling to inhibit prostate tumor formation and growth. J Biol Chem, 2003. 278(39): p. 37569-73.
Buchholz, M., et al., A multistep high-content screening approach to identify novel functionally relevant target genes in pancreatic cancer. PLoS One, 2015. 10(4): p. e0122946.
Campanile, A. and G. Iaccarino, G-protein-coupled receptor kinases in cardiovascular conditions: focus on G-protein-coupled receptor kinase 2, a gain in translational medicine. Biomark Med, 2009. 3(5): p. 525-40.
Chen, J.Y., et al., Paeoniflorin inhibits proliferation of fibroblast-like synoviocytes through suppressing G-protein-coupled receptor kinase 2. Planta Med, 2012. 78(7): p. 665-71.
Cho, M.C., et al., Enhanced contractility and decreased beta-adrenergic receptor kinase-1 in mice lacking endogenous norepinephrine and epinephrine. Circulation, 1999. 99(20): p. 2702-7.
Davio, C.A., et al., H1 and H2 histamine receptors in N-nitroso-N-methylurea (NMU)-induced carcinomas with atypical coupling to signal transducers. Biochem Pharmacol, 1995. 50(1): p. 91-6.
Dhami, G.K., et al., Phosphorylation-independent regulation of metabotropic glutamate receptor signaling by G protein-coupled receptor kinase 2. J Biol Chem, 2002. 277(28): p. 25266-72.
Dicker, F., et al., Phosphorylation-independent inhibition of parathyroid hormone receptor signaling by G protein-coupled receptor kinases. Proc Natl Acad Sci U S A, 1999. 96(10): p. 5476-81.
Diviani, D., et al., Effect of different G protein-coupled receptor kinases on phosphorylation and desensitization of the alpha1B-adrenergic receptor. J Biol Chem, 1996. 271(9): p. 5049-58.
Fernandez N, Monczor F, Lemos B, Notcovich C, Baldi A, Davio C, Shayo C. Reduction of G protein-coupled receptor kinase 2 expression in U-937 cells attenuates H2 histamine receptor desensitization and induces cell maturation. Mol Pharmacol. 62(6):1506-1514. 2002
Fernandez N, Monczor F, Baldi A, Davio C, Shayo C. Histamine H2 receptor trafficking. Role of arrestin, dynamin and clathrin in H2r internalization. Mol. Pharmacol. 74(4):1109-1118. 2008.
Fernandez N, Gottardo F, Alonso N, Monczor F, Shayo C, and Davio C. Roles of phosphorylation dependent and independent mechanisms in the regulation of Histamine H2 receptor by G Protein-coupled Receptor Kinase 2. J Biol Chem. 286(33):28697-706. 2011.
Freedman, N.J., et al., Phosphorylation and desensitization of human endothelin A and B receptors. Evidence for G protein-coupled receptor kinase specificity. J Biol Chem, 1997. 272(28): p. 17734-43.
Gartner, F., et al., Desensitization and internalization of endothelin receptor A: impact of G protein-coupled receptor kinase 2 (GRK2)-mediated phosphorylation. J Biol Chem, 2013. 288(45): p. 32138-48.
Han, CC et al., Regulatory effects of GRK2 on GPCRs and non-GPCRs and posible use as a drug target. Int J Mol Med, 2016. 38:987-994.
Homan, K.T., et al., Structural and functional analysis of g protein-coupled receptor kinase inhibition by paroxetine and a rationally designed analog. Mol Pharmacol, 2014. 85(2): p. 237-48.
Hullmann, J., et al., The expanding GRK interactome: Implications in cardiovascular disease and potential for therapeutic development. Pharmacol Res, 2016. 110: p. 52-64.
Iaccarino, G., et al., Reciprocal in vivo regulation of myocardial G protein-coupled receptor kinase expression by beta-adrenergic receptor stimulation and blockade. Circulation, 1998. 98(17): p. 1783-9.
Iwata, K., et al., Bimodal Regulation of the Human H1 Histamine Receptor by G Protein-coupled Receptor Kinase 2. Journal of Biological Chemistry, 2005. 280(3): p. 2197-2204.
Jaber, M., et al., Essential role of beta-adrenergic receptor kinase 1 in cardiac development and function. Proc Natl Acad Sci U S A, 1996. 93(23): p. 12974-9.
King, D.W., et al., Differential expression of GRK isoforms in nonmalignant and malignant human granulosa cells. Endocrine, 2003. 22(2): p. 135-42.
Koch, W.J., et al., Cardiac function in mice overexpressing the beta-adrenergic receptor kinase or a beta ARK inhibitor. Science, 1995. 268(5215): p. 1350-3.
Kong, G., R. Penn, and J.L. Benovic, A beta-adrenergic receptor kinase dominant negative mutant attenuates desensitization of the beta 2-adrenergic receptor. J Biol Chem, 1994. 269(18): p. 13084-7.
Lembo, P.M., M.H. Ghahremani, and P.R. Albert, Receptor selectivity of the cloned opossum G protein-coupled receptor kinase 2 (GRK2) in intact opossum kidney cells: role in desensitization of endogenous alpha2C-adrenergic but not serotonin 1B receptors. Mol Endocrinol, 1999. 13(1): p. 138-47.
Leosco, D., et al., Lymphocyte G-protein-coupled receptor kinase-2 is upregulated in patients with Alzheimer's disease. Neurosci Lett, 2007. 415(3): p. 279-82.
Lymperopoulos, A., et al., Adrenal GRK2 upregulation mediates sympathetic overdrive in heart failure. Nat Med, 2007. 13(3): p. 315-23.
Mak, J.C., et al., Increased expression of G protein-coupled receptor kinases in cystic fibrosis lung. Eur J Pharmacol, 2002. 436(3): p. 165-72.
Matkovich, S.J., et al., Cardiac-specific ablation of G-protein receptor kinase 2 redefines its roles in heart development and beta-adrenergic signaling. Circ Res, 2006. 99(9): p. 996-1003.
Mayor Menendez, F., El interactoma de la kinasa GRK2 y sus implicancias fisiopatológicas. Monografias de la Real Academia Nacional de Farmacia, 2009. XXIV.
Namkung, Y., et al., G protein-coupled receptor kinase-2 constitutively regulates D2 dopamine receptor expression and signaling independently of receptor phosphorylation. J Biol Chem, 2009. 284(49): p. 34103-15.
Nogues, L., et al., G Protein-coupled Receptor Kinase 2 (GRK2) Promotes Breast Tumorigenesis Through a HDAC6-Pin1 Axis. EBioMedicine, 2016. 13: p. 132-145.
Obrenovich, M.E., et al., Insights into cerebrovascular complications and Alzheimer disease through the selective loss of GRK2 regulation. J Cell Mol Med, 2009. 13(5): p. 853-65.
Oppermann, M., et al., Phosphorylation of the type 1A angiotensin II receptor by G protein-coupled receptor kinases and protein kinase C. J Biol Chem, 1996. 271(22): p. 13266-72.
Pearce, L.R., D. Komander, and D.R. Alessi, The nuts and bolts of AGC protein kinases. Nat Rev Mol Cell Biol, 2010. 11(1): p. 9-22.
Pierce, K.L., R.T. Premont, and R.J. Lefkowitz, Seven-transmembrane receptors. Nat Rev Mol Cell Biol, 2002. 3(9): p. 639-50
Rajagopal, S. and S.K. Shenoy, GPCR desensitization: Acute and prolonged phases. Cell Signal, 2017.
Reiter, E., et al., Kinase-inactive G-protein-coupled receptor kinases are able to attenuate follicle-stimulating hormone-induced signaling. Biochem Biophys Res Commun, 2001. 282(1): p. 71-8.
Sallese, M., et al., Selective regulation of Gq signaling by G protein-coupled receptor kinase 2: direct interaction of kinase N terminus with activated galphaq. Mol Pharmacol, 2000. 57(4): p. 826-31.
Schumacher, S.M., et al., Paroxetine-mediated GRK2 inhibition reverses cardiac dysfunction and remodeling after myocardial infarction. Sci Transl Med, 2015. 7(277): p. 277ra31.
Shayo C, Fernandez N, Legnazzi BL, Monczor F, Mladovan A, Baldi A, Davio C. Histamine H2 receptor desensitization: involvement of a select array of G protein-coupled receptor kinases. Mol Pharmacol. 60(5):1049-1056. 2001.
Sorriento, D., et al., "Freeze, Don't Move": How to Arrest a Suspect in Heart Failure - A Review on Available GRK2 Inhibitors. Frontiers in Cardiovascular Medicine, 2016. 3: p. 48.
Sterne-Marr, R., et al., Characterization of GRK2 RH domain-dependent regulation of GPCR coupling to heterotrimeric G proteins. Methods Enzymol, 2004. 390: p. 310-36.
Strasser, R.H., D.R. Sibley, and R.J. Lefkowitz, A novel catecholamine-activated adenosine cyclic 3',5'-phosphate independent pathway for beta-adrenergic receptor phosphorylation in wild-type and mutant S49 lymphoma cells: mechanism of homologous desensitization of adenylate cyclase. Biochemistry, 1986. 25(6): p. 1371-7.
Suo, Z., et al., Abnormality of G-protein-coupled receptor kinases at prodromal and early stages of Alzheimer's disease: an association with early beta-amyloid accumulation. J Neurosci, 2004. 24(13): p. 3444-52.
Tesmer, V.M., et al., Snapshot of activated G proteins at the membrane: the Galphaq-GRK2-Gbetagamma complex. Science, 2005. 310(5754): p. 1686-90.
Thal, D.M., et al., Paroxetine is a direct inhibitor of g protein-coupled receptor kinase 2 and increases myocardial contractility. ACS Chem Biol, 2012. 7(11): p. 1830-9.
Tian, X., et al., Effects of paroxetine-mediated inhibition of GRK2 expression on depression and cardiovascular function in patients with myocardial infarction. Neuropsychiatr Dis Treat, 2016. 12: p. 2333-2341.
Ungerer, M., et al., Altered expression of beta-adrenergic receptor kinase and beta 1-adrenergic receptors in the failing human heart. Circulation, 1993. 87(2): p. 454-63.
Usui, H., et al., RGS domain in the amino-terminus of G protein-coupled receptor kinase 2 inhibits Gq-mediated signaling. Int J Mol Med, 2000. 5(4): p. 335-40.
Wang, Q., et al., Paroxetine alleviates T lymphocyte activation and infiltration to joints of collagen-induced arthritis. Sci Rep, 2017. 7: p. 45364.Willets, J.M., et al., Imaging of muscarinic acetylcholine receptor signaling in hippocampal neurons: evidence for phosphorylation-dependent and -independent regulation by G-protein-coupled receptor kinases. J Neurosci, 2004. 24(17): p. 4157-62.
Zhang, Z., et al., Phosphorylation-independent desensitization of metabotropic glutamate receptor 5 by G protein-coupled receptor kinase 2 in HEK 293 cells. Mol Biol (Mosk), 2013. 47(1): p. 137-46.

## Claims

1. A compound of Formula I 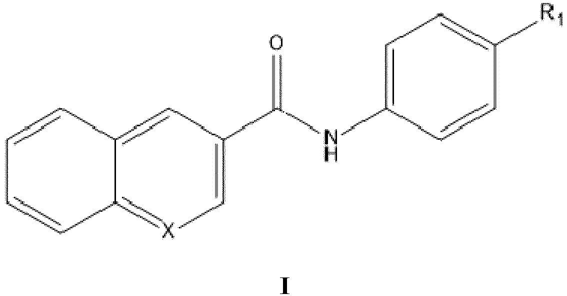
wherein **X** is an atom selected from carbon and nitrogen, and
**R₁** is selected from the groups methyl carboxylate and 1,3,4-oxadiazol-2-yl and wherein the compound is selected from methyl 4-(naphthalene-2-amido)benzoate and N-[4-(1,3,4-oxadiazol-2-yl)phenyl]quinoline-3-carboxamide.

2. A compound, according to claim 1, of Formula II

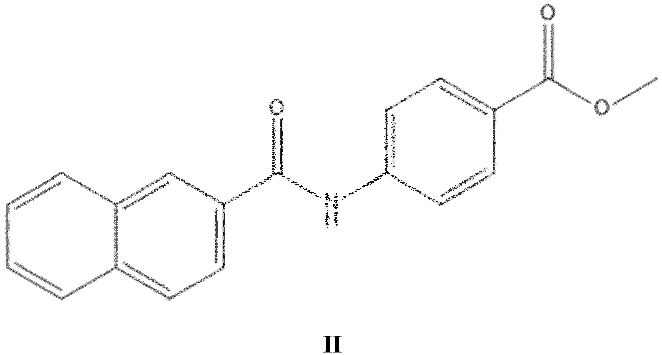

3. A compound, according to claim 1, of Formula III

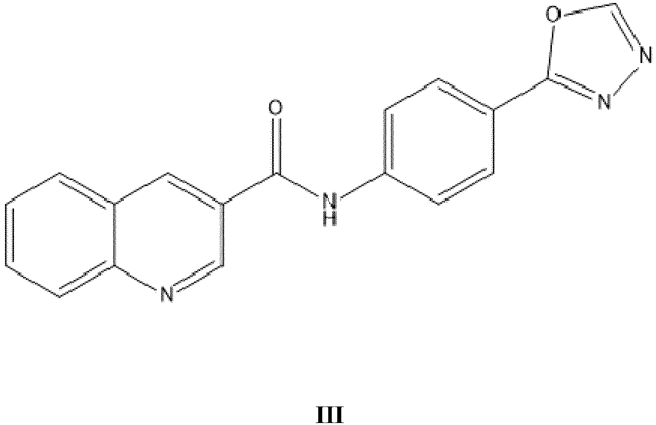

4. A pharmaceutical composition comprising at least a compound according to claim 1 and pharmaceutically acceptable excipients.

5. A pharmaceutical composition comprising a compound according to claim 2 and pharmaceutically acceptable excipients.

6. A pharmaceutical composition comprising a compound according to claim 3 and pharmaceutically acceptable excipients.

7. A pharmaceutical composition comprising at least a compound according to any one of claims 2 and 3 and an anti-tumor agent.

8. A pharmaceutical composition according to any one of claims 4 to 7, the composition being a pharmaceutical composition that may be administered via oral, parenteral or transdermal route.

9. A pharmaceutical composition according to any one of claims 4 to 7, the composition being useful for the treatment and/or control of cancer, hypertension, cardiac insufficiency, inflammatory diseases, Alzheimer's disease (AD), rheumatoid arthritis, cystic fibrosis (CF), sepsis and multiple sclerosis (MS).

10. A compound according to any of claims 1 to 3, for use in the treatment of a condition mediated by a GRK2 cell protein.

11. A compound according to any of claims 1 to 3, for use in the treatment and/or control of heart disease, cancer, inflammatory diseases, Alzheimer's disease (AD), rheumatoid arthritis, cystic fibrosis (CF), sepsis or multiple sclerosis (MS).

## Patentansprüche

1. Verbindung der Formel I, 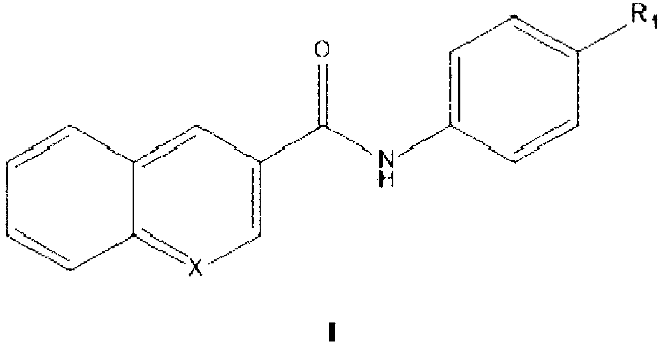
worin **X** ein Atom, ausgewählt aus Kohlenstoff und Stickstoff, ist, und
**R₁** aus den Gruppen Methylcarboxylat und 1,3,4-Oxadiazol-2-yl ausgewählt ist und worin die Verbindung aus Methyl-4-(naphtalene-2-amido)benzoat und N-[4-(1,3,4-oxadiazol-2-yl)phenyl]chinolin-3-carboxamid ausgewählt ist.

2. Verbindung nach Anspruch 1 der Formel II

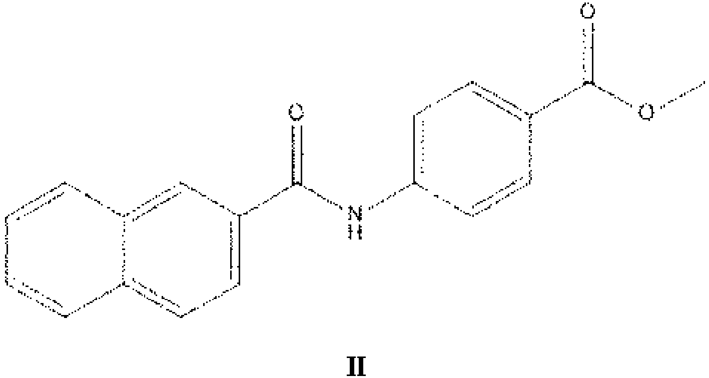

3. Verbindung nach Anspruch 1 der Formel III

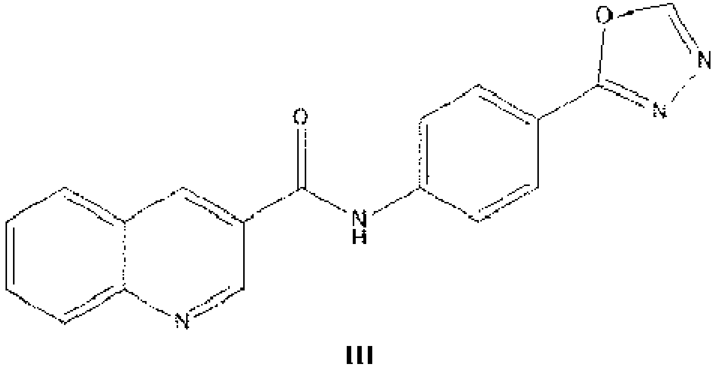

4. Pharmazeutische Zusammensetzung, umfassend mindestens eine Verbindung nach Anspruch 1 und einen pharmazeutisch akzeptablen Hilfsstoff.

5. Pharmazeutische Zusammensetzung, umfassend eine Verbindung nach Anspruch 2 und einen pharmazeutisch akzeptablen Hilfsstoff.

6. Pharmazeutische Zusammensetzung, umfassend eine Verbindung nach Anspruch 3 und einen pharmazeutisch akzeptablen Hilfsstoff.

7. Pharmazeutische Zusammensetzung, umfassend mindestens eine Verbindung nach einem der Ansprüche 2 und 3 und ein Antitumormittel.

8. Pharmazeutische Zusammensetzung nach einem der Ansprüche 4 bis 7, wobei die Zusammensetzung eine pharmazeutische Zusammensetzung ist, die auf oralem, pareteralem oder transdermalem Weg verabreicht werden kann.

9. Pharmazeutische Zusammensetzung nach einem der Ansprüche 4 bis 7, wobei die Zusammensetzung für die Behandlung und/oder Kontrolle von Krebs, Bluthochdruck, Herzinsuffizienz, entzündlichen Erkrankungen, Alzheimer-Krankheit (AD), rheumatoider Arthritis, zystischer Fibrose (CF), Sepsis und multipler Sklerose (MS) nützlich ist.

10. Verbindung nach einem der Ansprüche 1 bis 3 zur Verwendung bei der Behandlung eines durch GKR2-Zellprotein vermittelten Zustands.

11. Verbindung nach einem der Ansprüche 1 bis 3 zur Verwendung bei der Behandlung und/oder Kontrolle von Herzkrankheiten, Krebs, entzündlichen Erkrankungen, Alzheimer-Krankheit (AD), rheumatoider Arthritis, zystischer Fibrose (CF), Sepsis und multipler Sklerose (MS).

## Revendications

1. Composé de Formule I 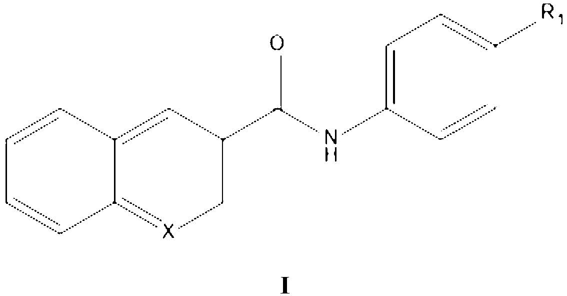
dans lequel **X** est un atome choisi parmi le carbone et l'azote, et
**R₁** est choisi parmi les groupes carboxylate de méthyle et 1,3,4-oxadiazol-2-yl et dans lequel le composé est choisi parmi le 4-(naphtalène-2-amido)benzoate de méthyle et le N-[4-(1,3,4-oxadiazol-2-yl)phényl]quinoléine-3-carboxamide.

2. Composé, selon la revendication 1, de Formule II

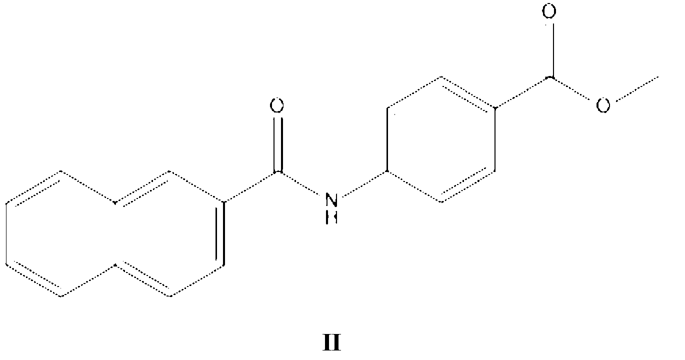

3. Composé, selon la revendication 1, de Formule III

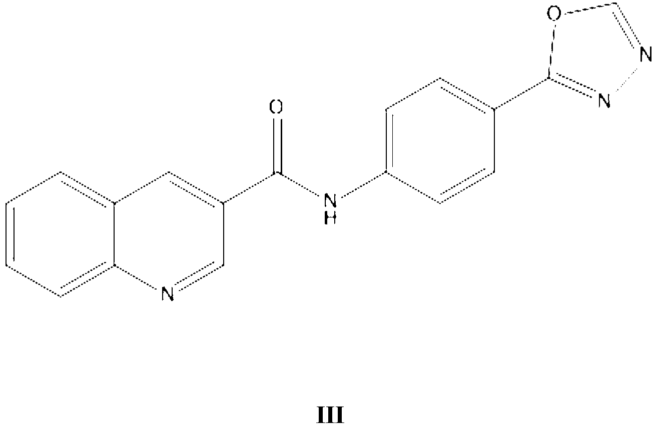

4. Composition pharmaceutique comprenant au moins un composé selon la revendication 1 et des excipients pharmaceutiquement acceptables.

5. Composition pharmaceutique comprenant un composé selon la revendication 2 et des excipients pharmaceutiquement acceptables.

6. Composition pharmaceutique comprenant un composé selon la revendication 3 et des excipients pharmaceutiquement acceptables.

7. Composition pharmaceutique comprenant au moins un composé selon l'une quelconque des revendications 2 et 3 et un agent antitumoral.

8. Composition pharmaceutique selon l'une quelconque des revendications 4 à 7, la composition étant une composition pharmaceutique pouvant être administrée par voie orale, parentérale ou transdermique.

9. Composition pharmaceutique selon l'une quelconque des revendications 4 à 7, la composition étant utile pour le traitement et/ou le contrôle du cancer, de l'hypertension, de l'insuffisance cardiaque, des maladies inflammatoires, de la maladie d'Alzheimer (MA), de la polyarthrite rhumatoïde, de la mucoviscidose (MV), du sepsis et de la sclérose en plaques (SEP).

10. Composé selon l'une des revendications 1 à 3, destiné à être utilisé dans le traitement d'une affection médiée par une protéine cellulaire GRK2.

11. Composé selon l'une des revendications 1 à 3, destiné à être utilisé dans le traitement et/ou le contrôle des maladies cardiaques, du cancer, des maladies inflammatoires, de la maladie d'Alzheimer (MA), de la polyarthrite rhumatoïde, de la mucoviscidose (MV), du sepsis et de la sclérose en plaques (SEP).
